# EUROPEAN PATENT SPECIFICATION

(11) **EP 2 155 696 B1**
(45) Date of publication and mention of the grant of the patent: **22.03.2017**
(21) Application number: 08750834.7
(22) Date of filing: 13.05.2008
(51) Int. Cl.: C07D 241/04, A61K 31/495, A61P 25/00

(54) **PIPERAZINE SALTS AS D3/D2 ANTAGONISTS**
PIPERAZINSALZE ALS D3/D2-ANTAGONISTEN
NOUVEAUX SELS DE PIPÉRAZINE COMME ANTAGONISTES DES RÉCEPTEURS DOPAMINERGIQUES D<SB>3</SB>/D<SB>2</SB>

(30) Priority: 11.05.2007 HU 0700339
(43) Date of publication of application: 24.02.2010
(73) Proprietor: Richter Gedeon Nyrt., 1103 Budapest (HU)
(72) Inventor: ÁGAINÉ CSONGOR, Éva, H-1031 Budapest (HU); CZIBULA, László, H-1103 Budapest (HU); SEBÖK, Ferenc, H-5800 Mezökovácsháza (HU); DOMÁNY, György, H-1022 Budapest (HU); GREINER, István, H-1021 Budapest (HU)
(74) Representative: Hoffmann Eitle
(86) International application number: PCT/HU2008/000044
(87) International publication number: WO 2008/139235

(56) References cited:
- WO-A-03/029233
- WO-A-2005/012266
- WO-A-2006/082456

## Description

### FIELD OF THE INVENTION

The present invention relates to novel monohydrochloride salts of *trans* 4-{2-[4-(2,3-dichlorophenyl)-piperazine-1-yl]-ethyl}-N,N-dimethylcarbamoyl-cyclohexylamine, to processes for their preparation to their use in the treatment and/or prevention of conditions which require modulation of dopamine receptors and to pharmaceutical compositions containing them.

### BACKGROUND OF THE INVENTION

Hungarian Patent Specification No. P0302451 discloses (thio)-carbamoyl-cyclohexane derivatives that are D₃/D₂ dopamin receptor subtype preferring ligands. One particular compound disclosed therein is *trans* 4-{2-[4-(2,3-dichlorophenyl)-piperazine-1-yl]-ethyl}-N,N-dimethylcarbamoyl-cyclohexylamine acts as dopamine receptor antagonist, particularly D₃/D₂ receptor antagonists and are useful in the treatment and prevention of pathological conditions which require modulation of dopamine receptors.

It is a general requirement for pharmaceutical compositions that an active agent present in the composition exhibits suitable physical, physicochemical and chemical properties. One important property for the active agent is its solubility, particularly its solubility in water. When the active agent has insufficient solubility in water, the agent typically must be converted into a form which has the appropriate solubility properties, for example, a salt and/or a solvate of the active agent. However, in the case of salts and/or solvates , only pharmaceutically acceptable salts and/or solvates of the active agent may be used in the preparation of pharmaceutical compositions.

Moreover, for successful industrial scale synthesis, the active agent must possess properties that make it easy to handle and produce on a large scale. In many cases, a crude active agent product is recovered from a reaction mixture by a complicated and often multistep process, which can decrease yields and cause a substantial increase in production costs. Therefore, also for economic reasons, the active agent must be easy-to handle and readily isolable. The handling properties also have a considerable effect on the resulting purity of the active agent, which is one of the most important factors to be considered in the pharmaceutical industry. Another matter of great importance is the stability of the form of the active agent used. The durability of a pharmaceutical composition, which includes the stability of the active agent itself, is of great importance, especially for quality control purposes.

It is well known that these properties of an active agent may be improved by choosing an appropriate salt form thereof. However, selection of a suitable salt for a particular active agent is not always straightforward, since the properties of salts of different compounds formed with the same salt forming agent may differ greatly

The base form of the salts according to the invention, namely the *trans* 4- {2-[4-(2,3-dichlorophenyl)-piperazine-1-yl]-ethyl} -N,N-dimethylcarbamoyl-cyclohexylamine base is substantially insoluble in water. Therefore our aim was to provide a compound form of the *trans* 4-{2-[4-(2,3-dichlorophenyl)-piperazine-1-yl]-ethyl}-N,N-dimethylcarbamoyl-cyclohexylamine which meets all the above mentioned solubility, handling and stability requirements.

### Brief description of the invention.

In the course of our experiments we have surprisingly found, that among the numerous salts described in the technical field the monohydrochloride salts exhibit excellent stability, isolability handling and solubility properties.

Accordingly, the present invention relates to novel monohydrochloride, salts of *trans* 4-{2-[4-(2,3-dichlorophenyl)-piperazine-1-yl]-ethyl}-N,N-dimethylcarbamoyl-cyclohexylamine and/or their hydrates and/or solvates, to processes for preparing them, to pharmaceutical compositions containing them and to their use in the therapy and/or prevention of conditions which require modulation of dopamine receptors, particularly D₃/D₂ receptors. Conditions which require modulation of dopamine receptor are for example psychotic states (e.g. schizophrenia, schizo-affective disorders, etc.), drug abuse (e.g. alcohol, cocaine, nicotine, opiate, etc. abuse), cognitive impairment accompanying schizophrenia, mild-to moderate cognitive deficits, dementia, psychotic states associated with dementia, eating disorders (e.g. bulimia nervosa, etc.), attention deficit disorders, infantile hyperactivity disorders, psychotic depression, mania, paranoid and delusional disorders, dyskinetic disorders (e.g. Parkinson's disease, neuroleptic induced parkinsonism, tardive dyskinesia), anxiety disorders, sexual functional disorders, sleeping disorders, emesis, aggression, autism.

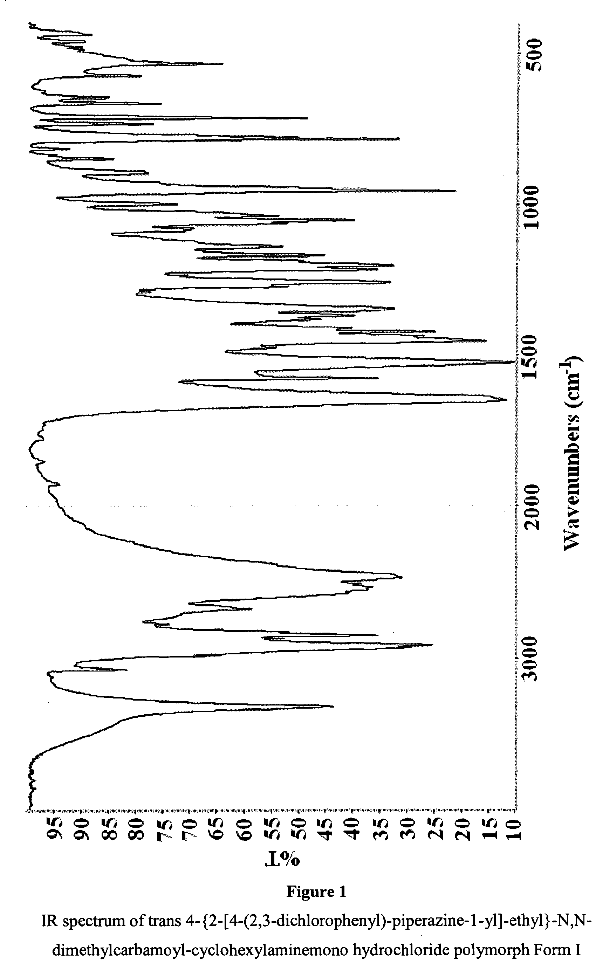

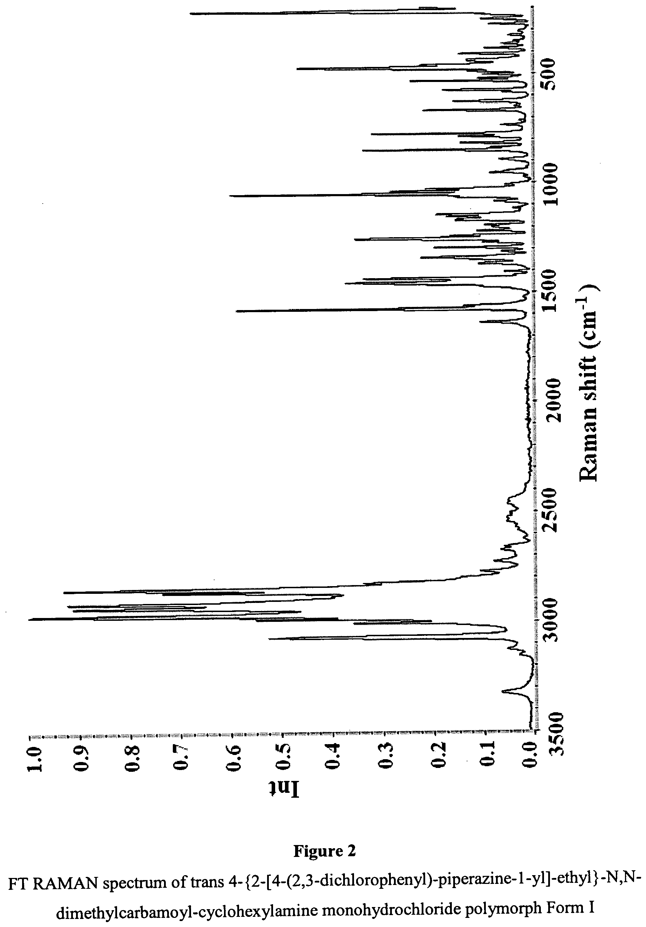

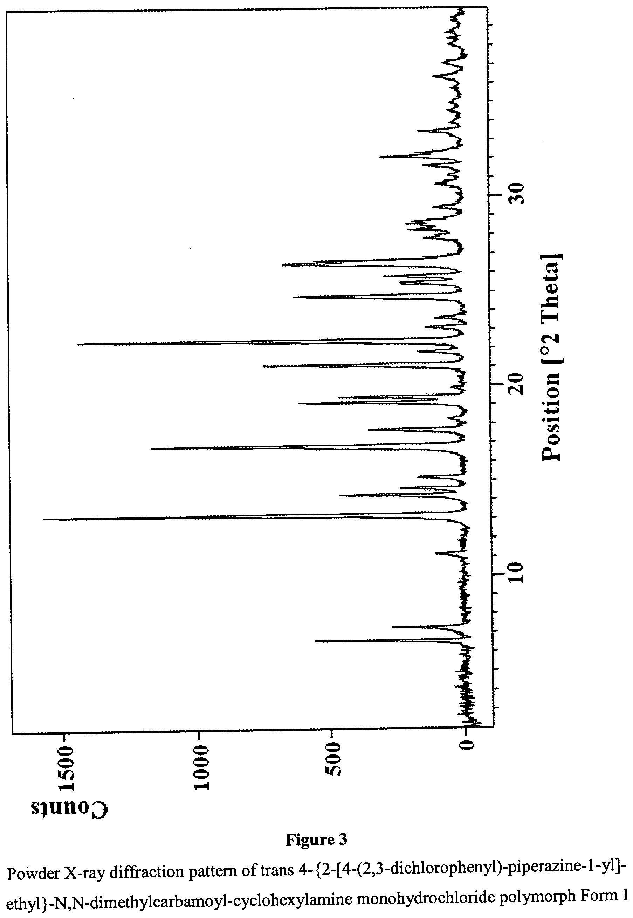

The present invention therefore relates to the subject matter as indicated in the below items 1 to 14.
Item 1. *Trans* 4-{2-[4-(2,3-dichlorophenyl)-piperazine-1-yl]-ethyl}-N,N-dimethylcarbamoyl-cyclohexylamine monohydrochloride and/or hydrates and/or solvates thereof.
Item 2. Process for the preparation of the compounds of item 1 characterized by that *trans* 4-{2-[4-(2,3-dichlorophenyl)-piperazine-1-yl]-ethyl}-N,N-dimethylcarbamoyl-cyclohexylamine base is suspended or dissolved in an appropriate solvent or mixture of solvents, then HCl, or a salt thereof prepared with a base which is weaker base than the *trans* 4-{2-[4-(2,3-dichlorophenyl)-piperazine-1-yl]-ethyl}-N,N-dimethylcarbamoyl-cyclohexylamine, or a solution thereof is added, and optionally the reaction mixture is concentrated and/or cooled, then the product obtained is isolated by filtration.
Item 3. Process according to item 2, wherein a salt of HCl prepared with a base which is weaker than the trans 4-{2-[4-(2,3-dichlorophenyl)-piperazine-1-yl]-ethyl}-N,N-dimethylcarbamoyl-cyclohexylamine base is used.
Item 4. A pharmaceutical composition comprising the compound according to item 1 and one or more pharmaceutically acceptable ingredient(s).
Item 5. The compound according to item 1 for use in the treatment and/or prevention of conditions, which require modulation of a dopamine receptor.
Item 6. The compound for use according to item 5, wherein the dopamine receptor is dopamine D₃ and/or dopamine D₂ receptor.
Item 7. The compound for use according to item 5, wherein the condition which requires modulation of dopamine receptor is selected from schizophrenia, schizo-affective disorders, cognitive impairment accompanying schizophrenia, mild-to moderate cognitive deficits, dementia, psychotic states associated with dementia, psychotic depression, mania, paranoid and delusional disorders, dyskinetic disorders (*e.g*. Parkinson's disease), neuroleptic induced parkinsonism, depression, anxiety and drug abuse.
Item 8. The compound according to item 1, which is in the form of crystalline trans 4-{2-[4-(2,3-dichlorophenyl)-piperazine-I-yl]-ethyl}-N,N-dimethylcarbamoyl-cyclohexylamine hydrochloride anhydrate (Form I).
Item 9. The crystalline form of item 8 wherein the infrared spectrum comprises characteristic peaks at 3321, 2931, 2914, 2466, 1652, 1526, 956, 784 and 715 cm⁻¹ ± 4 cm⁻¹.
Item 10. The crystalline form of item 8 having an infrared spectrum as depicted in Figure 1.
Item 1.. The crystalline form of item 8, wherein the Raman spectrum comprises characteristic peaks at 3070, 2986, 2969, 2933, 2914, 2864, 2850, 1578, 1458, 1052, and 475 cm⁻¹ ± 4 cm⁻¹_{.}
Item 12. The crystalline form of item 8 having a Raman spectrum as depicted in Figure 2.
Item 13. The crystalline form of item 8, wherein the powder X-ray diffraction pattern comprises peaks at 6.6, 7.3, 13.2, 14.2, 14.6, 16.9, 21.1, 22.4, 24.8, 26.5 and 26.6° ± 0.2 degrees 2θ.
Item 14. The crystalline form of item 8 having a powder X-ray diffraction pattern as depicted in Figure 3.

### Detailed description of the invention

The present invention relates to *trans* 4-{2-[4-(2,3-dichlorophenyl)-piperazine-1-yl]-ethyl}-N,N-dimethylcarbamoyl-cyclohexylamine monohydrochloride-salts and/or their hydrates and/or solvates.

Moreover, the present invention relates to processes for the preparation of *trans* 4-{2-[4-(2,3-dichlorophenyl)-piperazine-1-yl]-ethyl}-N,N-dimethylcarbamoyl-cyclohexylamine monohydrochloride salts and/or their hydrates and/or solvates.

The salts according to the invention can be prepared from the base form in the following manner: *trans* 4-{2-[4-(2,3-dichlorophenyl)-piperazine-1-yl]-ethyl}-N,N-dimethylcarbamoyl-cyclohexylamine base is dissolved or suspended in a suitable solvent or a mixture of solvents, then hydrochloric acid, or a salt of hydrochloric acid prepared by reaction of the acid with a base which is weaker base than *trans* 4-{2-[4-(2,3-dichlorophenyl)-piperazine-1-yl]-ethyl}-N,N-dimethylcarbamoyl-cyclohexylamine, or a solution thereof is added to the reaction mixture. Optionally, the salt form is isolated, (for example by concentrating the reaction mixture, or alternatively, by cooling the reaction mixture (with or without concentrating the mixture first) and isolating the resulting precipitate by filtration. In an exemplary preparation of *trans* 4-{2-[4-(2,3-dichlorophenyl)-piperazine-1-yl]-ethyl}-N,N-dimethylcarbamoyl-cyclohexylamine monohydrochloride, *trans* 4-{2-[4-(2,3-dichlorophenyl)-piperazine-1-yl]-ethyl}-N,N-dimethylcarbamoyl-cyclohexylamine is added to a mixture of methanol/water, then hydrochloric acid in 20-30 % excess of the stochiometric amount is added The reaction mixture then is heated to afford a homogenous solution and, after cooling, the product is isolated by filtration.
The monohydrochloride salts of *trans-*4-{2-[4-(2,3-dichlorophenyl)-piperazine-1-yl]-ethyl}-N,N-dimethylcarbamoyl-cyclohexyl-amine according to the invention exhibit excellent stability when stored as aqueous solutions.

The monohydrochloride salts of *trans*-4-{2-[4-(2,3-dichlorophenyl)-piperazine-1-yl]-ethyl}-N,N-dimethylcarbamoyl-cyclohexyl-amine also exhibit excellent stability when stored as in the solid state.

The monohydrochloride salts according to the invention can be well isolated and recovered in high purity also at industrial scale. Owing to their excellent stability, isolability, and purity properties the salts according to the invention are highly valuable for pharmaceutical use. The monohydrochloride salt may be prepared in the highest yield and highest purity. Another advantage of the monohydrochloride salt that it can readily be prepared using standard solvents and reaction conditions..

In another aspect, the present invention relates to the use of *trans* 4-{2-[4-(2,3-dichlorophenyl)-piperazine-1-yl]-ethyl}-N,N-dimethylcarbamoyl-cyclohexylamine monohydrochloride salts and/or their hydrates and/or solvates in the manufacture of a medicament for the treatment or prevent conditions which require modulation of dopamine receptors, especially dopamine D₃ and/or D₂ receptors.

Dysfunction of the dopaminergic neurotransmitter system can be observed in the pathology of several neuropsychiatric disorders such as schizophrenia, Parkinson's disease and the drug abuse. The effect of dopamine is mediated via at least five distinct dopamine receptors belonging to D₁-(i.e. D₁, and D₅), or D₂- (i.e. D₂, D₃ and D₄) receptor families. D₃ receptors have been shown to have characteristic distribution in the central dopaninergic systems. Namely, they were found in high densities in certain limbic structures, such as nucleus accumbens and islands of Calleya. Therefore, selective modulation of D₃ receptors may be a promising approach for more selective modulation of the dopaminerg functions and consequently offers successful therapeutic interventions in several abnormalities such as schizophrenia, emotional or cognitive dysfunctions (Sokoloff, P. et al: Nature, 1990, 347, 146; Schwartz, J.-C. et al.: Clin. Neuropharmacol., 1993, 16, 295; Levant, B.: Pharmacol. Rev., 1997, 49, 231), drug abuse (Pilla, C. et al: Nature, 1999, 400, 371) and Parkinson's disease (Levant, B. et al.: CNS Drugs, 1999, 12, 391) or pain (Levant, B. et al.: Neurosci. Lett., 2001, 303, 9).

The dopamine D₂ receptors are widely distributed in the brain and are known to be involved in numerous physiological functions and pathological states. Dopamine D₂ antagonists are for example used as antipsychotic agents. However, it is also well known that massive antagonism of the D₂ receptors leads to unwanted side effects, such as extrapyramidal motor symptoms, psychomotor sedation, or cognitive blunting. These side effects seriously restrict the therapeutic utilization of D2 antagonists (Wong A.H.C. et al.: Neurosci. Biobehav. Rev. 2003, 27, 269.).

The present invention provides the use of *trans* 4-{2-[4-(2,3-dichlorophenyl)-piperazine-1-yl]-ethyl}-N,N-dimethylcarbamoyl-cyclohexylamine monohydrochloride salts and/or their hydrates and/or solvates in the preparation of a medicament for the treatment of conditions which require modulation of dopamine D₃ and/or dopamine D₂ receptors. Such conditions which require modulation of dopamine D₃ and/or dopamine D₂ receptors are for example psychotic states (e.g. schizophrenia, schizo-affective disorders), cognitive impairment accompanying schizophrenia, mild-to moderate cognitive deficits, dementia, psychotic states associated with dementia, psychotic depression, mania, paranoid and delusional disorders, dyskinetic disorders (e.g. Parkinson's disease), neuroleptic induced parkinsonism, tardive dyskinesia, eating disorders (e.g. bulimia nervosa), attention deficit disorders, infantile hyperactivity disorders, anxiety, sexual functional disorders, sleeping disorder, emesis, aggression, autism and drug abuse.

The compounds of the present invention are for use in a method of treating conditions which require modulation of dopamine D₃ and/or D₂ receptors, for example psychotic states (e.g. schizophrenia, schizo-affective disorders), cognitive impairment accompanying schizophrenia, mild-to moderate cognitive deficits, dementia, psychotic states associated with dementia, psychotic depression, mania, paranoid and delusional disorders, dyskinetic disorders (e.g. Parkinson's disease), neuroleptic induced parkinsonism, tardive dyskinesia, eating disorders (e.g. bulimia nervosa), attention deficit disorders, infantile hyperactivity disorders, depression, anxiety, sexual functional disorders, sleeping disorders, emesis, aggression, autism and drug abuse, which comprises administering to a subject in need thereof an effective amount of *trans* 4-{2-[4-(2,3-dichlorophenyl)-piperazine-1-yl]-ethyl}-N,N-dimethylcarbamoyl-cyclohexylamine monohydrochloride, dihydrochloride, monohydrobromide, maleate and methanesulphonate salts and/or their hydrates and/or solvates.

A preferred use for D₃/D₂ ligands with D₃ preference according to the present invention relates to the treatment of schizophrenia, schizo-affective disorders), cognitive impairment accompanying schizophrenia, mild-to moderate cognitive deficits, dementia, psychotic states associated with dementia, psychotic depression, mania, paranoid and delusional disorders, dyskinetic disorders (e.g. Parkinson's disease), neuroleptic induced parkinsonism, depression, anxiety and drug abuse (e.g. cocaine, alcohol, nicotine abuse).

The particular combination of the two receptor-actions described above allows the simultaneous manifestations of the actions of D₃ functional antagonism (e.g. cognitive enhancer effect, inhibition of extrapyramidal motor symptoms, inhibitory action on drug abuse (and that of the D₂ functional antagonisms (e.g. antipsychotic effect). Furthermore, the same combination surprisingly results in cancelling the disadvantageous features of D₂ antagonism (e.g. extrapyramidal symptoms, psychomotor sedation and cognitive disturbances).

For use in medicine the monohydrochloride salts and/or their hydrates and/or solvates according to the invention are administered in the form of standard pharmaceutical compositions. Therefore, the present invention provides pharmaceutical compositions comprising *trans* 4-{2-[4-(2,3-dichlorophenyl)-piperazine-1-yl]-ethyl}-N,N-dimethylcarbamoyl-cyclohexylamine monohydrochloride, dihydrochloride, monohydrobromide, maleate and methanesulphonate salts and/or their hydrates and/or solvates and one or more pharmaceutically acceptable ingredients.

The monohydrochloride salts and/or their hydrates and/or solvates according to the invention may be administered by any convenient method, for example by oral, parenteral, buccal, sublingual, rectal or transdermal administration and the pharmaceutical compositions are adapted accordingly.

For oral administration *trans* 4-{2-[4-(2,3-dichlorophenyl)-piperazine-1-yl]-ethyl}-N,N-dimethylcarbamoyl-cyclohexylamine monohydrochloride salts and/or their hydrates and/or solvates can be formulated as liquids or solids, for example as syrups, suspensions, emulsions, tablets, capsules and lozenges.

A liquid formulation of the compounds of the present invention consists of a suspension or solution of the *trans* 4-{2-[4-(2,3-dichlorophenyl)-piperazine-1-yl]-ethyl}-N,N-dimethylcarbamoyl-cyclohexylamine monohydrochloride salts and/or their hydrates and/or solvates in a suitable liquid carrier(s), for example in an aqueous solvent such as water, ethanol or glycerol, or in a non-aqueous solvent, such as polyetileneglycol or an oil. The formulation may also contain one or more suspending agents, preservative, flavouring or colouring agent.

A composition in the solid form of a tablet can be prepared using any suitable pharmaceutical carrier(s) routinely used for preparing solid formulations. Examples of such carriers include magnesium stearate, starch, lactose, sucrose, cellulose, etc.

A composition in the solid form of a capsule can be prepared using routine encapsulation procedures. For example, pellets containing the active ingredient can be prepared using standard carriers and filed into a hard gelatine capsule; alternatively, a dispersion or suspension can be prepared using any suitable pharmaceutical carrier(s), for example aqueous gums, celluloses, silicates or oils and the dispersion or suspension then filled into a soft gelatine capsule.

Typical parenteral compositions consist of a solution or suspension of *trans* 4-{2-[4-(2,3-dichlorophenyl)-piperazine-1-yl]-ethyl}-N,N-dimethylcarbamoyl-cyclohexylamine monohydrochloride salts and/or their hydrates and/or solvates in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions of the present invention for nasal administration containing the salts and/or their hydrates and/or solvates may conveniently be formulated as aerosols, drops, gels and powders. Aerosol formulations of the present invention typically comprise a solution or fine suspension of *trans* 4-{2-[4-(2,3-dichlorophenyl)-piperazine-1-yl]-ethyl}-N,N-dimethylcarbamoyl-cyclohexylamine monohydrochloride. salts in a physiologically acceptable aqueous or non-aqueous solvent and are usually presented in a single quantity or multidose quantities in sterile sealed container, which can take the form of a cartridge or refill for use with an atomising device. Alternatively, the sealed container may be an unitary dispersing device, such as a single dose nasal inhaler fitted with a metering valve or an aerosol spray which is intended for disposal once and dropped when the contents of the container have been exhausted. When the dosage form comprises an aerosol spray, it will contain a propellant which can be compressed gas, such as compressed air or an organic propellant, such as a fluorochlorohydrocarbon. The aerosol spray dosage form can also take the form of a pump-atomiser. Compositions of the invention containing a salt according to the invention suitable for buccal or sublingual administration include tablets, lozenges and pastilles, wherein the active ingredient is formulated with a carrier, such as sugar and acacia, tragacanth, or gelatine and glycerol etc.

Compositions which contain the monohydrochloride salts and/or their hydrates and/or solvates according to the invention for rectal administration are conveniently in the form of suppositories containing a conventional suppository base, such as cocoa butter.

Compositions of the present invention containing the monohydrochloride. maleate and methanesulphonate salts of *trans* 4-{2-[4-(2,3-dichlorophenyl)-piperazine-1-yl]-ethyl}-N,N-dimethylcarbamoyl-cyclohexylamine and/or their hydrates and/or solvates according to the invention for transdermal administration include ointments, gels and patches.

Compositions of the present invention containing the *trans* 4-{2-[4-(2,3-dichlorophenyl)-piperazine-1-yl]-ethyl}-N,N-dimethylcarbamoyl-cyclohexylamine monohydrochloride salts and/or their hydrates and/or solvates according to the invention are preferably in a unit dose form, such as tablet, capsule or ampoule.

The invention is illustrated by the following examples. While the invention has been depicted and described by reference to exemplary embodiments of the invention, such a reference does not imply a limitation on the invention, and no such limitation is to be inferred. The invention is capable of considerable modification, alteration, and equivalents in form and function, as will occur to those ordinarily skilled in the pertinent arts having the benefit of this disclosure.

The depicted and described embodiments of the invention are exemplary only, and are not exhaustive of the scope of the invention.

### Reference Example 1

### Trans 4-{2-[4-(2,3-dichlorophenyl)-piperazine-1-yl]-ethyl}-N,N-dimethylcarbamoyl-cyclohexylamine methanesulfonate

3.0 g (0.007 mol) of *trans* 4-{2-[4-(2,3-dichlorophenyl):piperazine-1-yl]-ethyl}-N,N-dimethylcarbamoyl-eyclohexylamine and 0.46 ml (0.007 mol) of methanesulfonic acid were mixed in a mixture of 10 ml methanol and 80 ml acetonitrile. The reaction mixture was heated to boiling temperature and the homogenous solution obtained was concentrated to 25 ml by distillation. The resulting suspension was then stirred at a temperature between 0-5°C for 2 hours and the product was isolated by filtration.
In this manner 3.1 g of title compound was obtained.
Yield: 84 %.
Melting point: 225-229 °C.

### Reference Example 2

### Trans 4-{2-[4-(2,3-dichlorophenyl)-piperazine-1-yl]-ethyl}-N,N-dimethylcarbamoyl-cyclohexylamine maleate

3.0 g (0.007 mol) of *trans* 4-2-[4-(2,3-dichlorophenyl)-piperazine-1-yl]-ethyl}N,N-dimethylcarbamoyl-cyclohexylamin and 0.83 g (0.007 mol) of maleic acid were suspended in 150 ml of acetone. The reaction mixture was heated to boiling temperature and stirred for half an hour then concentrated to 25 ml by distillation. The resulting suspension was stirred at a temperature between 0-5 °C for 2 hours and the product obtained was isolated by filtration.
In this manner 3.14 g of title compound was obtained.
Yield: 82 %.
Melting point: 173-177 °C.

### Reference Example 3

### Trans 4-{2-[1-4-(2,3-dichlorophenyl)-piperazine-1-yl]-ethyl}-N,N-dimethylcarbamoyl-cyclohexylamine monohydrobromide

3.0 g (0.007 mol) of *trans* 4-{2-[4-(2,3-dichlorophenyl)-piperazine-1-yl]-ethyl}-N,N-dimethylcarbamoyl-cyclohexylamine was suspended in a mixture of 12 ml of methanol and 38 ml (1,5 %) of hydrogenbromide solution. The reaction mixture was heated to boiling temperature and the homogenous solution obtained was cooled to a temperature between 0-5 °C for 1 hour and was further stirred at the same temperature for 2 hours. The product obtained was isolated by filtration.
In this manner 3.0 g of title compound was obtained.
Yield: 85%.
Melting point: 248-252 °C.

### Reference Example 4

### Trans 4-{2-[4-(2,3-dichlorophenyl)-piperazine-1-yl]-ethyl}-N,N-dimethylcarbamoyl-cyclohexylamine dihydrochloride

3.0 g (0.007 mol) of *trans* 4-{2-[4-(2,3-dichlorophenyl)-piperazine-1-yl]-ethyl}-N,N-dimethylcarbamoyl-cyclohexylamine was suspended in 70 ml (20.5 g/100 ml) of anhydrous methanol saturated with hydrogenchloride. The reaction mixture was heated to boiling temperature and the resulting homogenous solution was concentrated to 25 ml by distillation. The suspension obtained was stirred at a temperature between 20-25°C for 2 hours and the product was isolated by filtration.
In this manner 3.0 g of title compound was obtained.
Yield: 85 %.
Melting point: 216-220 °C.

### Examples 1.

### Trans 4-{2-[4-(2,3-dichlorophenyl)-piperazine-1-yl]-ethyl}-N,N-dimethylcarbamoyl-cyclohexylamine monohydrochloride

147.5 g (0.345 mol) of *trans* 4-{2-[4-(2,3-dichlorophenyl)-piperazine-1-yl]-ethyl}-N,N-dimethylcarbamoyl-cyclohexylamine was suspended in a mixture of 300 ml of methanol and 1200 ml of distilled water. The reaction mixture was heated to a temperature between 60-75°C and a mixture of 40 ml (30%) of aqueous hydrogenchloride solution and 32 ml of water was added. The reaction mixture was stirred at a temperature between 60-75°C. The homogenous solution thus obtained was cooled to a temperature between 20-30°C for 1 hour then was further cooled to a temperature between 0-10°C and stirred at tis temperature for 3 hours. The product was isolated by filtration and washed with water.
In this manner 152.9 g of title compound was obtained.
Yield: 95 %.
Melting point: 221-224 °C.

The characterization of *trans* 4-{2-[4-(2,3-dichlorophenyl)-piperazine-1-yl]-ethyl}-N,N-dimethylcarbamoyl-cyclohexylamine monohydrochloride was carried out by thermogravimetric (TG), differential scanning colorimetric (DSC), infrared spectroscopic (FT-IR), Raman spectroscopic (FT-Raman) and powder X-ray diffraction (PXRD) solid phase analytical methods.

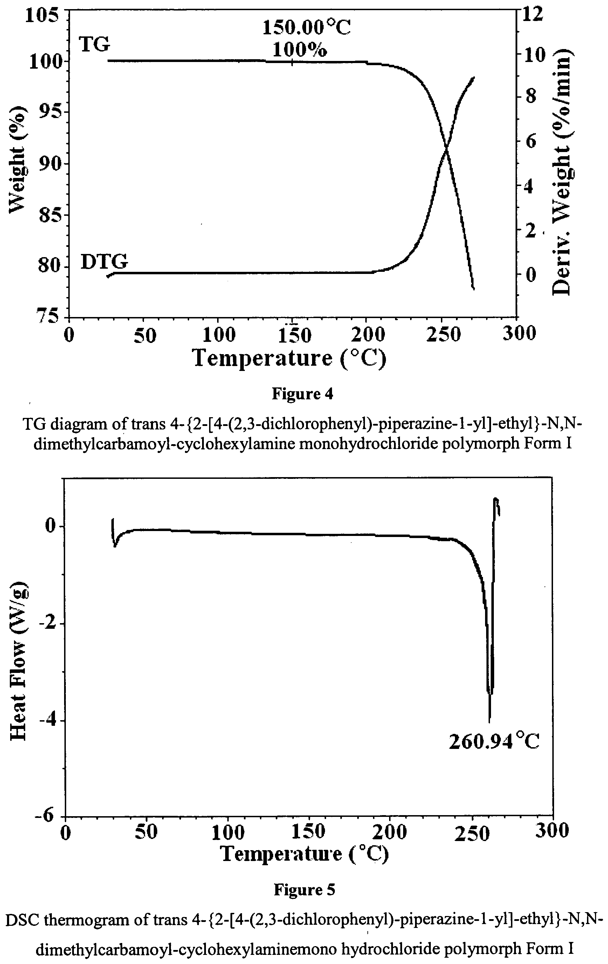

On the basis of TG and DSC measurements the *trans* 4-{2-[4-(2,3-dichlorophenyl)-piperazine-1-yl]-ethyl}-N,N-dimethylcarbamoyl-cyclohexylamine monohydrochloride salt was identified as a solvent-free and anhydrous form, which exhibits satisfactory thermal stability up to about 200°. After further heating *trans* 4-{2-[4-(2,3-dichlorophenyl)-piperazine-1-yl]-ethyl}-N,N-dimethylcarbamoyl-cyclohexylamine monohydrochloride salt melts above 220°C with accompanying simultaneously vigorous thermal decomposition and high weight loss, as it can be seen on the thermograms (Figures 4 and 5). The *trans* 4-{2-[4-(2,3-dichlorophenyl)-piperazine-1-yl]-ethyl}-N,N-dimethylcarbamoyl-cyclohexylamine monohydrochloride anhydrate polymorph Form I prepared according to the invention and its crystal structure can be fully identified and characterized by IR and Raman spectra and powder X-ray diffraction patterns (Figures 1, 2, and 3).

Parameters for solid phase analytical methods used are as follows.

### FT-IR spectroscopy

| | |
|---|---|
| Type of apparatus: | Thermo-Nicolet 6700 |
| Phase (solvent): | KBr |
| Spectral resolution | 4 cm⁻¹ |
| Scan-number: | 100 |

### FT-Raman spectroscopy

| | |
|---|---|
| Type of apparatus: | Thermo-Nicolet NXR9650 |
| Measuring range: | 3500 - 200 cm⁻¹ |
| Spectral resolution: | 4 cm⁻¹ |
| Scan-number: | 128 |
| Laser performance: | 300 mW |

### Powder X-ray diffraction

| | |
|---|---|
| Type of apparatus: | PANanalytical X'Pert PRO |
| Radiation: | CuK_{α} |
| Accelerating potential: | 40 kV |
| Anode current: | 40 mA |
| Goniometer: | PW3050/60 |
| Exposure speed: | 0,208 °2θ/s |
| Sample container: | Spinner PW3064 |
| Rotational speed of | |
| sample container: | 1 turn/s |
| Uncertainty of 2θ measurement: | ± 0,2° |

### TG analysis

| | |
|---|---|
| Type of apparatus: | TA Instruments TGA Q50 |
| Heating speed: | 10°C/min |
| Sample weight: | ∼5 - 10 mg |
| Atmosphere: | 60 ml/min N₂ |

### DSC analysis

| | |
|---|---|
| Type of apparatus: | TA Instruments DSC Q10 |
| Heating speed: | 10 °C/min |
| Sample weight: | ∼1 -2 mg |
| Pot type: | opened |
| Atmosphere: | 50 ml/min N₂ |

## Claims

1. *Trans* 4-{2-[4-(2,3-dichlorophenyl)-piperazine-1-yl]-ethyl}-N,N-dimethylcarbamoyl-cyclohexylamine monohydrochloride and/or hydrates and/or solvates thereof.

2. Process for the preparation of the compounds of claim 1 **characterized by** that *trans* 4-{2-[4-(2,3-dichlorophenyl)-piperazine-1-yl]-ethyl}-N,N-dimethylcarbamoyl-cyclohexylamine base is suspended or dissolved in an appropriate solvent or mixture of solvents, then HCl, or a salt thereof prepared with a base which is weaker base than the *trans* 4-{2-[4-(2,3-dichlorophenyl)-piperazine-1-yl]-ethyl}-N,N-dimethylcarbamoyl-cyclohexylamine, or a solution thereof is added, and optionally the reaction mixture is concentrated and/or cooled then the product obtained is isolated by filtration.

3. Process according to claim 2, wherein a salt of HCl prepared with a base which is weaker than the trans 4-{2-[4-(2,3-dichlorophenyl)-piperazine-1-yl]-ethyl}-N,N-dimethylcarbamoyl-cyclohexylamine base is used.

4. A pharmaceutical composition comprising the compound according to claim 1 and one or more pharmaceutically acceptable ingredient(s).

5. The compound according to claim 1 for use in the treatment and/or prevention of conditions, which require modulation of a dopamine receptor.

6. The compound for use according to claim 5, wherein the dopamine receptor is dopamine D₃ and/or dopamine D₂ receptor.

7. The compound for use according to claim 5, wherein the condition which requires modulation of dopamine receptor is selected from schizophrenia, schizo-affective disorders, cognitive impairment accompanying schizophrenia, mild-to moderate cognitive deficits, dementia, psychotic states associated with dementia, psychotic depression, mania, paranoid and delusional disorders, dyskinetic disorders (e.g. Parkinson's disease), neuroleptic induced parkinsonism, depression, anxiety and drug abuse.

8. The compound according to claim 1, which is in the form of crystalline trans 4-{2-[4-(2,3-dichlorophenyl)-piperazine-1-yl]-ethyl}-N,N-dimethylcarbamoyl-cyclohexylamine hydrochloride anhydrate (Form I).

9. The crystalline form of claim 8 wherein the infrared spectrum comprises characteristic peaks at 3321, 2931, 2914, 2466, 1652, 1526, 956, 784 and 715 cm⁻¹ ± 4 cm⁻¹.

10. The crystalline form of claim 8 having an infrared spectrum as depicted in Figure 1.

11. The crystalline form of claim 8, wherein the Raman spectrum comprises characteristic peaks at 3070, 2986, 2969, 2933, 2914, 2864, 2850, 1578, 1458, 1052, and 475 cm⁻¹ ± 4 cm⁻¹.

12. The crystalline form of claim 8 having a Raman spectrum as depicted in Figure 2.

13. The crystalline form of claim 8, wherein the powder X-ray diffraction pattern comprises peaks at 6.6, 7.3, 13.2, 14.2, 14.6, 16.9, 21.1, 22.4, 24.8, 26.5 and 26.6° ± 0.2 degrees 2θ.

14. The crystalline form of claim 8 having a powder X-ray diffraction pattern as depicted in Figure 3.

## Patentansprüche

1. *Trans*-4-{2-[4-(2,3-Dichlorphenyl)-piperazin-1-yl]-ethyl}-N,N-dimethylcarbamoyl-cyclohexylamin-Monohydrochlorid und/oder Hydrate und/oder Solvate davon.

2. Verfahren zur Herstellung der Verbindungen aus Anspruch 1, dadurch charakterisiert, dass die *trans*-4-{2-[4-(2,3-Dichlorphenyl)-piperazin-1-yl]-ethyl}-N,N-dimethylcarbamoyl-cyclohexylamin-Base in einem geeigneten Lösungsmittel oder einer Mischung von Lösungsmitteln suspendiert oder gelöst wird, dann HCl oder ein Salz davon, hergestellt mit einer Base, die eine schwächere Base als *trans*-4-{2-[4-(2,3-Dichlorphenyl)-piperazin-1-yl]-ethyl}-N,N-dimethylcarbamoyl-cyclohexylamin ist, oder eine Lösung davon hinzugefügt wird und optional die Reaktionsmischung konzentriert wird und/oder gekühlt wird, dann das erhaltene Produkt durch Filtration isoliert wird.

3. Verfahren gemäß Anspruch 2, wobei ein Salz von HCl, hergestellt mit einer Base, die eine schwächere Base als *trans*-4-{2-[4-(2,3-Dichlorphenyl)-piperazin-1-yl]-ethyl}-N,N-dimethylcarbamoyl-cyclohexylamin ist, verwendet wird.

4. Pharmazeutische Zusammensetzung, die die Verbindung gemäß Anspruch 1 und einen oder mehrere pharmazeutisch annehmbare(n) Inhaltsstoff(e) umfasst.

5. Verbindung gemäß Anspruch 1 zur Verwendung bei der Behandlung und/oder Vorbeugung von Zuständen, die die Modulation eines Dopamin-Rezeptors erfordern.

6. Verbindung zur Verwendung gemäß Anspruch 5, wobei der Dopamin-Rezeptor der Dopamin D₃-und/oder Dopamin D₂-Rezeptor ist.

7. Verbindung zur Verwendung gemäß Anspruch 5, wobei der Zustand, der die Modulation eines Dopamin-Rezeptors erfordert, ausgewählt ist aus Schizophrenie, schizoaffektive Störungen, Schizophrenie-begleitende kognitive Beeinträchtigung, milde bis moderate kognitive Defizite, Demenz, Demenz-assoziierte psychotische Zustände, psychotische Depression, Wahn, paranoide und wahnhafte Störungen, dyskinetische Störungen (z.B. Morbus Parkinson), neuroleptisch induzierter Parkinsonismus, Depression, Angst und Drogenmissbrauch.

8. Verbindung gemäß Anspruch 1, die in Form des wasserfreien kristallinen *trans*-4-{2-[4-(2,3-Dichlorphenyl)-piperazin-1-yl]-ethyl}-N,N-dimethylcarbamoyl-cyclohexylamin-hydrochlorids (Form I) vorliegt.

9. Kristalline Form gemäß Anspruch 8, wobei das Infrarotspektrum charakteristische Peaks bei 3321, 2931, 2914, 2466, 1652, 1526, 956, 784 und 715 cm⁻¹ ±4 cm⁻¹ umfasst.

10. Kristalline Form gemäß Anspruch 8, die ein Infrarotspektrum wie in Figur 1 dargestellt aufweist.

11. Kristalline Form gemäß Anspruch 8, wobei das Ramanspektrum charakteristische Peaks bei 3070, 2986, 2969, 2933, 2914, 2864, 2850, 1578, 1458, 1052 und 475 cm⁻¹ ±4 cm⁻¹ umfasst.

12. Kristalline Form gemäß Anspruch 8, die ein Ramanspektrum wie in Figur 2 dargestellt aufweist.

13. Kristalline Form gemäß Anspruch 8, wobei das Pulver-Röntgenbeugungsmuster Peaks bei 6,6, 7,3, 13,2, 14,2, 14,6, 16,9, 21,1, 22,4, 24,8, 26,5 und 26,6° ±0,2 Grad 2*θ* umfasst.

14. Kristalline Form gemäß Anspruch 8, die ein Pulver-Röntgenbeugungsmuster wie in Figur 2 dargestellt aufweist.

## Revendications

1. Monohydrochlorure de *trans* 4-{2-[4-(2,3-dichlorophényl)-pipérazine-1-yl]-éthyl}-N,N-diméthylcarbamoyl-cyclohexylamine et/ou des hydrates et/ou des sovates de celui-ci.

2. Procédé de préparation de composés selon la revendication 1, **caractérisé en ce que** la base de *trans* 4-{2-[4-(2,3-dichlorophényl)-pipérazine-1-yl]-éthyl}-N,N-diméthylcarbamoyl-cyclohexylamine est mise en suspension ou est dissoute dans un solvant ou dans un mélange de solvants approprié , puis du HCl, ou un sel correspondant préparé avec une base qui est une base plus faible que la *trans* 4-{2-[4-(2,3-dichlorophényl)-pipérazine-1-yl]-éthyl}-N,N-diméthylcarbamoyl-cyclohexylamine, ou une solution de celui-ci est ajoutée, et optionnellement le mélange réactionnel est concentré et/ou refroidi, puis le produit obtenu est isolé par filtration.

3. Procédé selon la revendication 2, dans lequel un sel de HCl préparé avec une base qui est plus faible que la *trans* 4-{2-[4-(2,3-dichlorophényl)-pipérazine-1-yl]-éthyl}-N,N-diméthylcarbamoyl-cyclohexylamine est utilisé.

4. Composition pharmaceutique comprenant le composé selon la revendication 1 et un ou plus d'un ingrédient(s) acceptable(s) au point de vue pharmaceutique.

5. Composé selon la revendication 1 destiné à être utilisé dans le traitement et/ou la prévention d'états qui nécessitent une modulation d'un récepteur dopaminergique.

6. Composé destiné à être utilisé selon la revendication 5, dans lequel le récepteur dopaminergique est un récepteur dopaminergique D₃ et/ou un récepteur dopaminergique D₂.

7. Composé destiné à être utilisé selon la revendication 5, dans lequel l'état qui nécessite une modulation d'un récepteur dopaminergique est choisi parmi la schizophrénie, les troubles schizo-affectifs, la déficience cognitive accompagnant la schizophrénie, les déficiences cognitives faibles à modérées, la démence, les états psychotiques associés à la démence, la dépression psychotique, la manie, les troubles paranoïdes ou de délire, les trouble dyskinétiques (par exemple la maladie de Parkinson), le parkinsonisme induit par les neuroleptiques, la dépression, l'anxiété et l'abus de drogues.

8. Composé selon la revendication 1, qui est sous la forme d'un anhydrate d'hydrochlorure de *trans* 4-{2-[4-(2,3-dichlorophényl)-pipérazine-1-yl]-éthyl}-N,N-diméthylcarbamoyl-cyclohexylamine cristallin (Forme I).

9. Forme cristalline selon la revendication 8, dans laquelle le spectre infrarouge comprend des pics caractéristiques à 3 321, 2 931, 2 914, 2 466, 1652, 1526, 956, 784 et 715 cm⁻¹ ± 4 cm⁻¹.

10. Forme cristalline selon la revendication 8, présentant un spectre infrarouge tel que représenté sur la Figure 1.

11. Forme cristalline selon la revendication 8, dans laquelle le spectre Raman comprend des pics caractéristiques à 3 070, 2 986, 2 969, 2 933, 2 914, 2 864, 2 850, 178, 1458, 1 052 et 475 cm⁻¹ ± 4 cm⁻¹.

12. Forme cristalline selon la revendication 8, présentant un spectre Raman tel que représenté sur la Figure 2.

13. Forme cristalline selon la revendication 8, dans laquelle le diagramme de diffraction des rayons X sur poudre comprend les pics à 6,6, 7,3, 13,2, 14,2, 14,6, 16,9, 21,1, 22,4, 24,8, 26,5 et 26,6° ± 0,2 degrés (2θ).

14. Forme cristalline selon la revendication 8, présentant un diagramme de diffraction des rayons X sur poudre tel que représenté sur la Figure 3.
